(19) Europäisches Patentamt
European Patent Office
Office européen des brevets

(11) Publication number: 0 001 845
A2

(12) EUROPEAN PATENT APPLICATION

(21) Application number: 78101338.8

(22) Date of filing: 09.11.78

(51) Int. Cl.$^2$: C 07 C 103/52
A 61 K 37/02

(30) Priority: 09.11.77 US 849830

(43) Date of publication of application: 16.05.79 Bulletin 79/10

(84) Designated contracting states: BE CH DE FR GB LU NL SE

(71) Applicant: Merck & Co., Inc.
Patent Department 126 East Lincoln Avenue
Rahway, N.J. 07065(US)

(72) Inventor: Veber, Daniel Frank
290 Batleson Road
Ambler Pennsylvania 19002(US)

(72) Inventor: Hirschmann, Ralph Franz
740 Palmer Place
Blue Bell Pennsylvania 19422(US)

(72) Inventor: Nutt, Ruth Foelsche
Hill Road RD 1
Green Lane Pennsylvania 18054(US)

(74) Representative: Abitz, Walter, Dr.-Ing. et al,
Abitz, Morf, Gritschneder P.O.Box 860109
D-8000 München 86(DE)

(54) **Analeptic tripeptides, process for their preparation and pharmaceutical composition containing the same.**

(57) Novel tripeptides related to thyrotropin releasing hormone, with methylprolineamide or methylthioprolineamide as the C-terminal amino acid, are stimulants of the central nervous system. They are preparable by standard peptide synthetic procedures.

EP 0 001 845 A2

# ANALEPTIC TRIPEPTIDES, PROCESS FOR THEIR PREPARATION AND PHARMACEUTICAL COMPOSITION CONTAINING THE SAME

## Background of the Invention

This invention is concerned with the tripeptides L-2-ketopiperidine-6-carbonyl-L-histidyl-*trans*-3-methyl-L-prolineamide, L-2-ketopiperidine-6-carbonyl-L-histidyl-5R-methylthiazolidine-4R-carboxamide, L-pyroglutamyl-L-histidyl-5R-methylthiazolidine-4R-carboxamide or pharmaceutically acceptable salts thereof which are useful as central nervous system stimulants with a rapid onset of action and a minimum of adverse side effects.

Thyrotropin releasing hormone (TRH) is a naturally-occurring, well-known tripeptide, L-pyroglutamyl-L-histidyl-L-prolinamide. Besides its hormone releasing activity it is also known to have antidepressant activity.

Recently several publications have reported the preparation of derivatives and analogs of TRH with a view to augmenting the antidepressant activity of TRH. Veber et al., in U.S. Patent 3,959,248 describes a group of compounds analagous to TRH which manifests an increased antidepressant activity and/or decreased hormone releasing activity. Belgian Patent No. 832,783 describes a TRH derivative in which the amide nitrogen of prolineamide, is substituted with a 2-carbamylethyl

group resulting in the tetrapeptide amide, L-pyroglutamyl-L-histidyl-L- prolyl-β-alaninamide which also manifests an increased antidepressant activity relative to the hormone releasing activity. Also German Patent 2,609,154 describes the TRH analog wherein the proline of the TRH has been replaced by trans-3-methyl-L-proline, among others. This analog, too, has an augmented CNS stimulant activity.

The novel compounds of this invention display greater activity as central nervous system stimulants than does TRH. Accordingly it is an object of this invention to provide the novel compounds, means for their syntheses, pharmaceutical compositions employing the novel compounds as active ingredient, and a method of producing central nervous stimulation.

Detailed Description of the Invention

The novel compounds of this invention have structural formula:

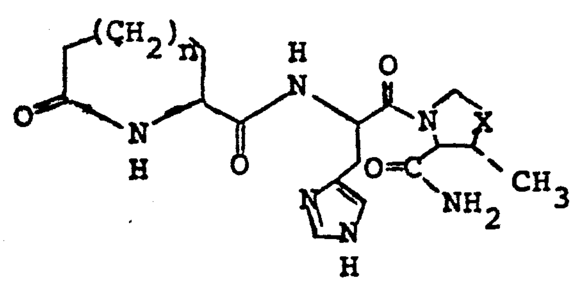

or a pharmaceutically acceptable salt thereof, wherein, each amino acid has the L-configuration, X is $-CH_2-$ or -S- and the methyl and carboxamide groups of that amino acid are trans with respect to one another, n is 0 or 1 when X is -S- and n is 1 when X is $-CH_2-$.

To facilitate discussion, the tripeptides of this invention wherein X is $-CH_2-$ and -S- will be designated respectively as

L-HPCA-L-His-L-3-MePro-$NH_2$,

L-HPCA-L-His-L-5-MeThioPro-$NH_2$, and

L-PCA-L-His-L-5-MeThioPro-$NH_2$,

wherein:

L-HPCA- is L-2-ketopiperidine-6-carbonyl, 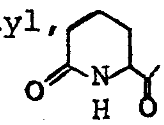

L-His- is L-histidyl, 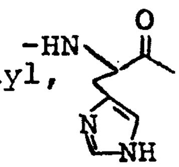

L-3-MePro- is trans-3-methyl-L-prolyl, 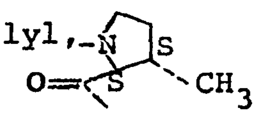

L-5-MeThioPro is trans-5-methyl-L-thioprolyl,

or 5R-methylthiazolidine-4R-carboxyl, 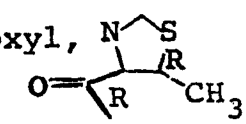

and,

L-PCA- is L-pyroglutamyl, 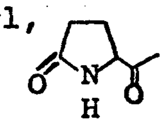

The pharmaceutically acceptable salts contemplated to be within the scope of the present invention are those prepared from inorganic or organic acids known in the art to provide pharmaceutically acceptable salts, such as hydrochloric, sulfuric, hydrobromic, phosphoric, tartaric, fumaric, citric, malic, maleic, ascorbic, acetic, lactic, oleic, pamoic, palmitic, isethionic acid,

pyroglutamic acid or the like.

The novel process of the present invention comprises formation of an amide, or peptide bond between the carboxyl group of one moiety of the tripeptide and the amino group of another moiety as illustrated by the following equations:

1) L-HPCA-OH + L-His-L-3-MePro-$NH_2$
2) L-HPCA-L-His-OH + L-3-MePro-$NH_2$
3) L-HPCA-L-His-L-3-MePro-OH + $NH_3$

In each of the above reactions and following discussion, the use of L-3-MePro- is illustrative only and it is understood that L-5-MeThioPro- may replace it. In that case, L-PCA may replace L-HPCA.

It is also understood, that, although not shown above, the amino acid or peptide starting materials may have amino blocking groups to protect those amino groups not intended to take part in peptide bond formation. For example, the histidine group (L-His) has an imidazole nitrogen which may be protected from participation in peptide bond formation. Many suitable protective groups are recognized in the art for this purpose, a common one being 2,4-dinitrophenyl (DNP), tosyl or benzyl. Similarly, the ring nitrogen of L-HPCA (L-2-keto-piperidine-6-carbonyl) may be protected by an art recognized blocking group, but in this instance is usually not required. Other common amino blocking groups employed in peptide chemistry, particularly for protection of the α-amino group, are the benzyloxycarbonyl, or t-butyloxycarbonyl (Boc) group.

Peptide bond formation may be accomplished in any one of several methods falling into two general classes:

1) direct coupling of a carboxyl and an amino group; or

2) coupling of a carboxyl derivative with an amino group.

The direct coupling generally requires a coupling agent to promote the condensation such as dicyclohexylcarbodiimide, either alone or in combination with 1-hydroxy-benzotriazole; triphenylphosphite and imidazole; triphenylphosphine and dipyridyl-2, 2'-disulfide; or Woodward's reagent; or diphenylphosphorylazide. In the second case useful carboxyl derivatives are azides, halides, mixed anhydrides, or active esters such as p-nitrophenyl, N-hydroxysuccinimidyl, perfluorophenyl, cyanomethyl, p-nitrophenylthio, and the like.

The coupling is normally conducted in an anhydrous inert organic solvent such as dimethyl formamide or chlorinated hydrocarbons such as chloroform, methylene chloride, 1,2-dichloroethane, or the like, at temperatures from about 0°C. to about 25°C.

The reaction represented by Equation 1) supra is particularly suitable for preparing the novel compound of the present invention. The reaction comprises mixing the dipeptide, L-His-L-3-MePro-$NH_2$ with from 1-1.5 equivalents of L-HPCA-OH, at least one equivalent of N-hydroxybenzotriazole and at least one equivalent of dicyclohexylcarbodiimide in methylene chloride at room temperature and allowing the reaction to proceed for about 0.5 to about 5 hours. It is preferred that the L-His imidazole nitrogen be protected with a DNP group which is finally removed by treatment with mercaptoethanol at 10-30°C. for 10 minutes to about 1 hour.

The novel compounds of the present invention are central nervous system stimulants and may be used for treating mammals suffering from central nervous system depression. They may be administered by any convenient method, e.g. orally, parenterally, intravenously, sublingually, by insulfation, by suppository, or the like.

Dosage levels adequate to produce the desired effect are used. Generally, the dosage range will be from 0.05 to 100 mg. per dose, administered alone, or in combination with accepted pharmaceutical carriers.

Example 1

L-2-ketopiperidine-6-carbonyl-L-histidyl-trans-3-methyl-L-prolineamide (L-HPCA-L-His-L-3-MePro-$NH_2$)

Step A: Preparation of Boc(DNP)-L-His-hydroxysuccinimide ester

A solution of 16.8 g. (40 mmol) of Boc(DNP)-L-His and 4.6 g. (40 mmol) of N-hydroxysuccinimide in 100 ml. of THF was treated at 0°C with 9.07 g. (44 mmol) of DCCI for 2 hours. The reaction mixture was filtered, the precipitate washed three times with THF, and the filtrate evaporated to a foamy residue and triturated once with ether and precipitated once with cyclohexane from $CH_2Cl_2$, to remove residual DCCI. The crude product was dissolved in $CHCl_2$, the solution extracted three times with dilute bicarbonate solution, dried over $MgSO_4$, filtered, and the filtrate evaporated to an oil. Trituration with ether gave 14.7 g. of amorphous product. The IR spectrum of the product in $CHCl_3$ was consistent with the desired active ester structure.

Step B: Preparation of Boc(DNP)-L-His-L-3-MePro-OH

A suspension of 2 g. (15.5 mmol) of trans-3-methyl-L-proline, 8.74 g. (17.06 mmol) of Boc(DNP)-L-His-hydroxysuccinimide ester in 38 ml. $CH_2Cl_2$ was neutralized to pH 7.0-7.2 (moist pH paper, range 6-8) with a total of 4 ml. (27.6 mmol) of triethylamine and stirred magnetically for 3.5 hours. The solution was extracted with three portions of dilute aqueous citric acid and the $CH_2Cl_2$ layer was dried over $MgSO_4$, filtered and evaporated to 8.65 g. of crude product.

Step C: Preparation of Boc(DNP)-L-His-L-3-MePro-$NH_2$

A suspension of 5.32 g. (10 mmol) Boc(DNP)-L-His-L-3-MePro-OH, 1.62 g. (12 mmol) of anhydrous hydroxybenzotriazole, 2.67 g. (50 mmol) of $NH_4Cl$ in 50 ml. $CH_2Cl_2$ was stirred magnetically and treated with 1.35 ml. (9.3 mmol) of triethylamine and 2.06 g. (10 mmol) of DCCI. An additional 1.75 ml. of triethylamine was added portionwise over 20 minutes to keep the pH (moist pH paper, range 6-8) of the reaction at 7.0-7.2. After 4.5 hours at room temperature, the reaction mixture was filtered and the filtrate extracted once with water, four times with dilute aqueous citric acid, three times with aqueous bicarbonate, dried over $MgSO_4$, filtered, and evaporated *in vacuo*. The residue was precipitated twice from $CH_2Cl_2$ with petroleum ether to give 4.8 g. of dipeptideamide.

Step D: Preparation of (DNP)-L-His-L-3-MePro-$NH_2 \cdot 2HCl$

A solution of 2.0 g. (3.77 mmol) of Boc(DNP)-L-His-L-3-MePro-$NH_2$ in 40 ml. of ethylacetate was cooled in a -30°C isopropanol - dry ice bath and treated for 5 minutes with a stream of HCl gas, and at 0°C with a vigorous stream of $N_2$ for 5 minutes. Ether (~100 ml.) was added, and the suspension filtered, the solid product was washed with ether and dried in vacuo to give 1.75 g. deblocked dipeptide product.

Step E: Preparation of L-HPCA-L-His-L-3-MePro-$NH_2$

To a suspension of 93.3 mg. (.185 mmol) of (DNP)-L-His-L-3-MePro-$NH_2 \cdot 2HCl$, 29.4 mg. (.204 mmol), of L-HPCA, 29.6 mg. (.222 mmol) of anhydrous hydroxybenzotriazole in 1.5 ml. of $CH_2Cl_2$ was added 57.2 mg. (.278 mmol) of DCCI as a 0.5 ml. $CH_2Cl_2$ solution and 0.05 ml. (0.345 mmol) of triethylamine. An additional 0.035 ml. of triethylamine was added over 10 minutes to adjust the pH of the reaction to 7.2 (moist pH paper, range 6-8). After 15 minutes at room temperature, a thin layer chromatogram of the reaction showed absence of dipeptide starting material.

After 30 minutes at room temperature the tripeptide product solution was treated with 0.2 ml. of mercaptoethanol for 1 hour to remove the DNP blocking group. The reaction mixture was filtered and the filtrate was extracted 7 times with water ( total ~25 ml. $H_2O$),

the combined aqueous extracts were filtered through a 5 ml. Dowex 1 x 2 ($Ac^-$) column to remove L-HPCA and HBT, the filtrate was evaporated in vacuo to a small volume and freeze-dried to give 180 mg. of crude product. The product was chromatographed on a 40 g. silica gel column packed in 80-20-2 (by volume) ($CHCl_3$-MeOH- con. $NH_4OH$) and eluted with the same solvent at a rate of 20 ml./min. Fractions (20 ml.) 17-24 were combined, evaporated to a small volume and freeze-dried to give 40.6 mg. of single spot product,

rf 0.19, 80-20-2 ($CHCl_3$-MeOH-$H_2O$)
rf 0.28, 80-20-2 ($CHCl_3$-MeOH-$NH_4OH$)
rf 0.52, 30-20-4-6 ($CHCl_3$-MeOH-HOAc-$H_2O$)

$[\alpha]_D^{25}$ -54.3° (c 0.1, 50% HOAc)

Acid hydrolysis amino acid analysis:

His 0.96
$NH_3$ 1.01
α-$NH_2$adipic acid 1.05
3-MePro .99

An additional 19 mg. of product was obtained from side cuts of the silica column.

Employing the procedure of Example 1, Steps A through E, but substituting for the L-3-MePro-OH used in Step B thereof an equimolecular amount of L-5-MeThioPro-OH, there is produced L-2-ketopiperidine-6-carbonyl-L-histidyl-trans-5-methyl-L-thioprolineamide (L-HPCA-L-His-L-5-MeThio-Pro-$NH_2$).

rf 0.21, 80-20-2 ($CHCl_3$-MeOH-$H_2O$)
rf 0.27, 80-20-2 ($CHCl_3$-MeOH-$NH_4OH$)

Similarly using the procedure of Example there is produced L-pyroglutamyl-L-histidyl-trans-5-methyl-L-thioprolineamide (L-PCA-L-His-L-5-MeThioPro-$NH_2$) when L-5-MeThio-Pro-OH is used in place of L-3-MePro-OH in Step B and L-pyroglutamic acid, (L-PCA), is used in place of L-HPCA in Step E.

WHAT IS CLAIMED IS:

1. A process for the preparation of the compound of formula:

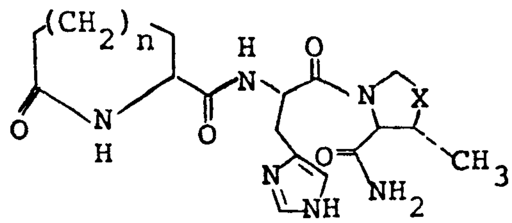

which comprises the formation of a peptide bond between:

(1) 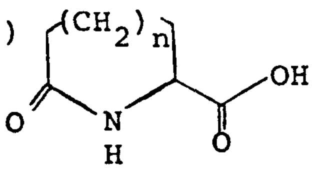 and 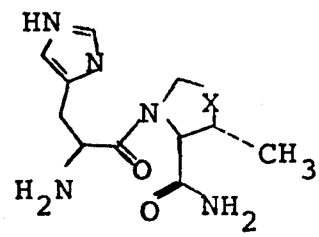

(2) 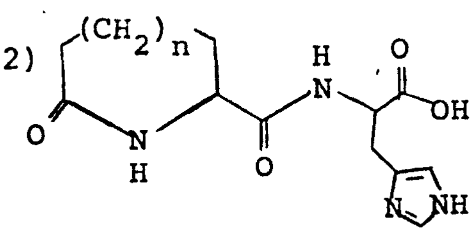 and 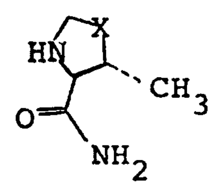 , or

(3) 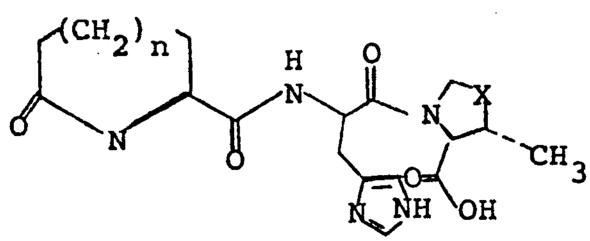 and $NH_3$

wherein each amino acid has the L-configuration; X is $-CH_2-$ or -S-; the $-CH_3$ and carboxamide group are

trans- with respect to one another n is 0 or 1 when X is -S- and n is 1 when X is $-CH_2-$; followed by removal of blocking groups if necessary.

2. The process of Claim 1 which comprises the formation of a peptide bond between

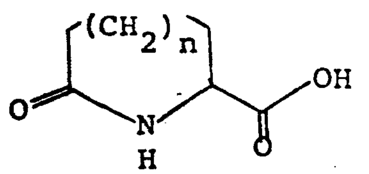 and 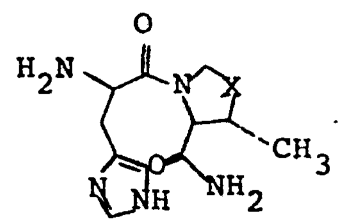

3. The process of Claim 2 which comprises the formation of a peptide bond between

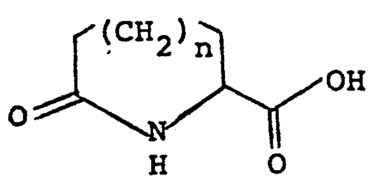

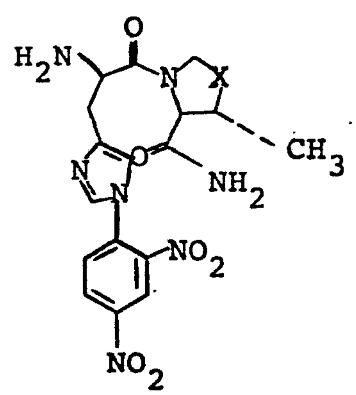

followed by removal of the DNP protecting group.

4. The process of Claim 3 wherein X is $-CH_2-$.

5. The process of Claim 3 wherein X is -S-.

6. A compound of structural formula:

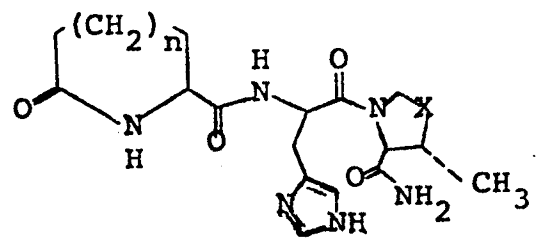

or a pharmaceutically acceptable salt thereof wherein each amino acid has the L-configuration; X is $-CH_2-$ or -S-; the $-CH_3$ and carboxamide group are trans; n is 0 or 1 when X is -S- and n is 1 when X is $-CH_2-$.

7. The compound of Claim 6 or a pharmaceutically acceptable salt thereof, wherein X is $-CH_2-$.

8. The compound of Claim 6 or a pharmaceutically acceptable salt thereof, wherein X is -S-.

9. A pharmaceutical composition for use as a CNS stimulant comprising a pharmaceutical carrier and an effective amount of a compound of formula:

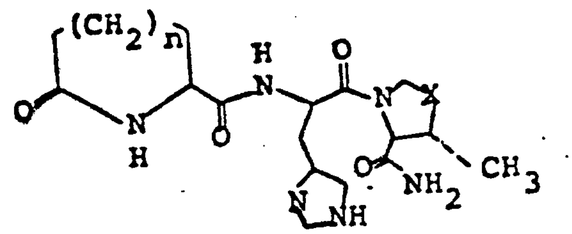

or a pharmaceutically acceptable salt thereof, wherein each amino acid has the L-configuration; X is $-CH_2-$ or -S-; the $-CH_3$ and carboxamide group are trans; n is 0 or 1 when X is -S- and n is 1 when X is $-CH_2-$.